# EUROPEAN PATENT APPLICATION

(11) **EP 4 660 621 A1**
(43) Date of publication of application: **10.12.2025**
(21) Application number: 23919874.0
(22) Date of filing: 01.11.2023
(51) Int. Cl.: G01N 27/12

(54) **METAL OXIDE SEMICONDUCTOR GAS SENSOR**

(30) Priority: 30.01.2023 US 202363441971 P
(71) Applicant: TDK Corporation, Tokyo 103-6128 (JP)
(72) Inventor: ISHIKURA, Tomokazu, Tokyo 103-6128 (JP); FUJITA, Kazutaka, Tokyo 103-6128 (JP); MASAOKA, Raitaro, Tokyo 103-6128 (JP)
(74) Representative: Epping - Hermann - Fischer
(86) International application number: PCT/JP2023/039455
(87) International publication number: WO 2024/161739

(57) **Abstract**

The present invention pertains to a metal oxide semiconductor gas sensor having a first electrode, a second electrode, and a sensitive layer in contact with both the first electrode and the second electrode. The sensitive layer includes In₂O₃ and Co₃O₄. The average porosity in a cross section of the sensitive layer is 15.7-22.0%. The proportion of In₂O₃ and the proportion of Co₃O₄ in a region excluding pores of the sensitive layer are 65-99.5 vol% and 0.5-35 vol%, respectively.

## Description

### Technical Field

The present invention relates to a metal oxide semiconductor gas sensor.

### Background

Because carbon monoxide (CO) or nitrogen dioxide (NO₂) causes addiction or air pollution, its concentration needs to be monitored, which requires a small, highly sensitive sensor. In particular, carbon monoxide (CO) is an incomplete combustion product and poisons a catalyst using high toxicity or as an impurity in hydrogen fuel. Thus, a high-performance sensor is required.

JP Patent No. 6586694 describes a gas sensor material for CO detection using an A₂₋ₓCeₓBO₄ compound. However, a sensor described in this publication has insufficient selectivity or sensitivity to a CO gas, and further improvement is demanded.

### Summary of Invention

### Problem to be solved by Invention

It is an object of an exemplary embodiment of the present invention to provide a metal oxide semiconductor gas sensor capable of detecting carbon monoxide (a CO gas) contained at a low concentration in a gas of interest.

### Means for solving the Problem

To achieve the above object, a metal oxide semiconductor gas sensor of an exemplary embodiment of the present invention includes a first electrode, a second electrode, and a sensing layer in contact with both the first electrode and the second electrode,
wherein
the sensing layer contains In₂O₃ and Co₃O₄,
the sensing layer has an average porosity of 15.7% or more and 22.0% or less in a section of the sensing layer,
In₂O₃ constitutes 65 vol% or more and 99.5 vol% or less of a region of the sensing layer excluding pores, and
Co₃O₄ constitutes 0.5 vol% or more and 35 vol% or less of the region.

A metal oxide semiconductor gas sensor of another exemplary embodiment of the present invention includes a first electrode, a second electrode, and a sensing layer in contact with both the first electrode and the second electrode,
wherein
the sensing layer contains a first metal oxide of an n-type semiconductor and a second metal oxide of a p-type semiconductor, and
the sensing layer has an average porosity of 15.7% or more and 22.0% or less in a section of the sensing layer.

φf - φn ≥ 1.0 may be satisfied, where φn (%) denotes an average porosity of a region closest to the first electrode and the second electrode, and φf (%) denotes an average porosity of a region farthest from the first electrode and the second electrode, provided that the sensing layer is divided into three regions in a thickness direction of the sensing layer.

The sensing layer may have an average pore size of 80 nm or more and 120 nm or less in a section of the sensing layer.

The sensing layer may have a thickness of 1.0 um or more.

### Brief Description of the Drawings

[FIG. 1] FIG. 1 is a plan view of a metal oxide semiconductor gas sensor of an embodiment of the present invention with a sensing layer being omitted.
[FIG. 2] FIG. 2 is a plan view of the metal oxide semiconductor gas sensor of the embodiment of the present invention.
[FIG. 3] FIG. 3 is a sectional view along a line A-A' of the metal oxide semiconductor gas sensor shown in FIG. 2.

### Detailed Description of Invention

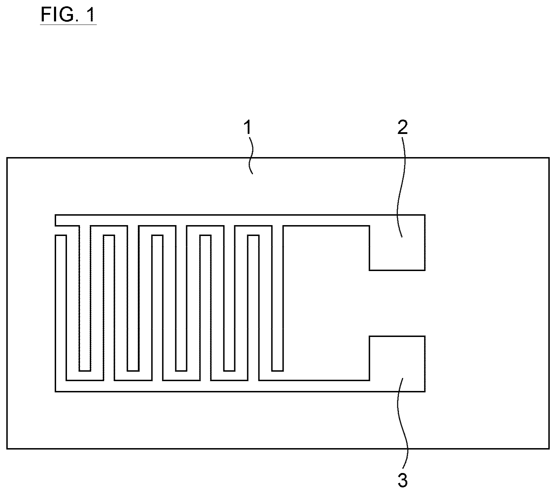

Hereinafter, a metal oxide semiconductor gas sensor 10 according to an embodiment of the present invention is described with reference to FIGS. 1 to 3.

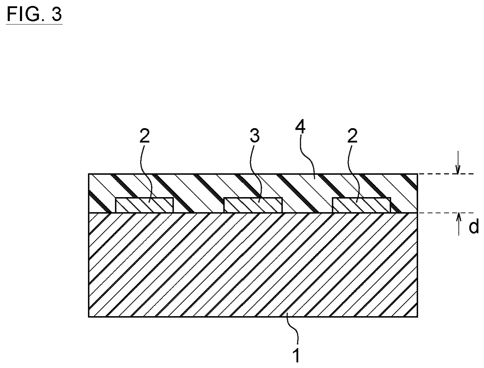

As shown in FIGS. 1 and 3, the metal oxide semiconductor gas sensor 10 includes a substrate 1 and a first electrode 2 and a second electrode 3 on the substrate 1. In the present embodiment, the first electrode 2 and the second electrode 3 are in an interdigitated shape, facing each other at a predetermined distance; however, the shape is not limited as long as the first electrode 2 and the second electrode 3 face each other at a predetermined distance. As shown in FIGS. 1 to 3, a sensing layer 4 is in contact with both the first electrode 2 and the second electrode 3 so as to cover portions of these electrodes 2 and 3 other than their lead-out electrode portions. The sensing layer 4 is provided on a surface of the substrate 1 having the first electrode 2 and the second electrode 3.

The sensing layer 4 is composed of a metal oxide material. The sensing layer 4 has pores, though their illustration is omitted in FIG. 3 showing a section of the sensing layer 4, so as to have an average porosity of 15.7% or more and 22.0% or less, preferably 15.9% or more and 22.0% or less, or more preferably 15.9% or more and 20.8% or less. Methods of measuring the average porosity are not limited.

For example, the ratio of the total area of the pores in at least one section of the sensing layer 4 to the entire area of the at least one section may be deemed to be the average porosity. The lower size limit of the pores regarded as such is, for example, 3 nm or more. Note that, for calculation of the average, the at least one section of the sensing layer 4 preferably has an area within a predetermined range in which at least one hundred pores are observed. Methods of observing the at least one section are not limited. Observation is carried out using a method in which the metal oxide material and the pores can be distinguished. Observation of the at least one section of the sensing layer 4 using, for example, a SEM, an optical microscope, or the like enables calculation of the average porosity.

Methods of determining the at least one section of the sensing layer 4 are not limited provided that the average porosity of the sensing layer 4 is appropriately measured. The total area percentage of the pores in, for example, a section parallel to the thickness direction of the sensing layer 4 is calculated. The average porosity is preferably found by averaging a measurement of a region in contact with the substrate 1 and a measurement of a region on the surface side externally exposed along the thickness direction of the sensing layer 4.

The sensing layer 4 contains at least In₂O₃ and Co₃O₄. In₂O₃ constitutes 65 vol% or more and 99.5 vol% or less of a region of the sensing layer 4 excluding the pores. Co₃O₄ constitutes 0.5 vol% or more and 35 vol% or less of that region.

The average porosity of the sensing layer 4 and the percentage of each component being within the predetermined ranges enable the metal oxide semiconductor gas sensor with high sensitivity to a CO gas and a short response time to be provided. Also, it is assumed that appropriate control of the average porosity of the sensing layer 4 enables the CO gas to readily enter the pores, oxygen adsorbed on a surface of the oxides to readily react with the gas, and a space charge barrier to more readily be lowered. It is assumed that this decreases resistance, further improves sensitivity, and contributes to a decrease in response time.

The percentage of In₂O₃ in the region of the sensing layer 4 excluding the pores is preferably 66.5 vol% or more and 99.4 vol% or less or is more preferably 88.5 vol% or more and 98.8 vol% or less. The percentage of Co₃O₄ in the region of the sensing layer 4 excluding the pores is preferably 0.6 vol% or more and 33.5 vol% or less or is more preferably 1.2 vol% or more and 11.5 vol% or less. Such ranges enable a decrease in response time and, in particular, improvement of sensitivity.

The percentage of In₂O₃ in the region of the sensing layer 4 excluding the pores is, in other words, the percentage of In₂O₃ in the metal oxide material constituting the sensing layer 4. The percentage of Co₃O₄ in the region of the sensing layer 4 excluding the pores is, in other words, the percentage of Co₃O₄ in the metal oxide material constituting the sensing layer 4. Note that the first electrode 2 and the second electrode 3 are not included in the sensing layer 4.

Methods of measuring the percentage of In₂O₃ and the percentage of Co₃O₄ are not limited. For measurement, for example, LA-ICP-MS or the like may be used.

The sensing layer 4 has an average pore size of preferably 80 nm or more and 120 nm or less. Methods of measuring the average pore size are not limited. For example, equivalent circle diameters of the pores in a section parallel to the thickness direction of the sensing layer 4 are calculated and averaged. The average pore size of the sensing layer 4 can be defined as the average of sizes of all pores observed in a section of the sensing layer 4 having an area within a predetermined range. The lower size limit of the pores regarded as such is similar to that of the average porosity.

Note that, for calculation of the average pore size, the area of the section of the sensing layer 4 within the predetermined range is preferably similar to that of the average porosity. That is, the section of the sensing layer 4 preferably has an area within the predetermined range in which at least one hundred pores are observed. Also, for calculation of the average pore size, a measurement of the region in contact with the substrate 1 and a measurement of the region on the surface side externally exposed along the thickness direction of the sensing layer 4 are preferably averaged. At the time of calculation of the average pore size, pores having a size of less than 80 nm or above 120 nm may be counted; however, such out-of-range pores are preferably 10% or less of all the observed pores in terms of number.

The average pore size is preferably determined based on mean free path of CO (90 to 130 nm). The average pore size being within the above range further readily increases sensitivity to a CO gas and further readily decreases response time.

The thickness of the sensing layer 4 is not limited. The thickness may be, for example, 1.0 um or more. The thickness of the sensing layer 4 being 1.0 um or more readily improves sensitivity to a CO gas. There is no upper limit of the thickness of the sensing layer 4. The upper limit may be, for example, 20 um or less. Note that, in a situation where the first electrode 2 and the second electrode 3 are embedded in the sensing layer 4 as shown in FIG. 3, the thickness of the sensing layer 4 denotes its thickness of a portion excluding the first electrode 2 and the second electrode 3. Specifically, a thickness d in FIG. 3 denotes the thickness of the sensing layer 4.

φf - φn ≥ 1.0 is preferably satisfied, where φn (%) denotes the average porosity of a region closest to the first electrode 2 and the second electrode 3, and φf (%) denotes the average porosity of a region farthest from the first electrode 2 and the second electrode 3, provided that the sensing layer 4 is trisected in the thickness direction. That is, in the sensing layer 4, the portion with which a gas of interest mainly comes into contact (outer surface side of the sensing layer 4) and its vicinity preferably have a high average porosity. Satisfaction of φf - φn ≥ 1.0 further readily increases sensitivity to a CO gas and further readily decreases response time.

Methods of measuring φf and φn are not limited. For example, an image, observed using a SEM or the like, of a section that is parallel to the thickness direction of the sensing layer 4 and does not include the first electrode 2 or the second electrode 3 may be trisected in the thickness direction. The total area percentage of the pores in a part of the image corresponding to the region closest to the first electrode 2 and the second electrode 3 (region closest to the substrate 1) may be defined as φn. The total area percentage of the pores in the region farthest from the first electrode 2 and the second electrode 3 may be defined as φf.

The percentage of In₂O₃ in the region closest to the first electrode 2 and the second electrode 3 excluding the pores may be 65 vol% or more and 99.5 vol% or less. The percentage of Co₃O₄ in that region excluding the pores may be 0.5 vol% or more and 35 vol% or less. Moreover, the percentage of In₂O₃ in the region farthest from the first electrode 2 and the second electrode 3 excluding the pores may be 65 vol% or more and 99.5 vol% or less. The percentage of Co₃O₄ in that region excluding the pores may be 0.5 vol% or more and 35 vol% or less. Note that the percentage of In₂O₃ and the percentage of Co₃O₄ (with the pores being excluded) in the metal oxide material constituting the sensing layer 4 may be analyzed by extracting a predetermined volume ratio (analyzable volume ratio) of the sensing layer 4. In this situation as well, the respective regions provide similar results.

The sensing layer 4 may further contain other metal oxide and/or SiO₂ to the extent that the sensitivity to a CO gas and response time are not significantly affected. As an example of the other metal oxide, TiO₂ or the like is mentioned. The other metal oxide and SiO₂ may constitute 10 vol% or less, in total, of the region of the sensing layer 4 excluding the pores.

The sensing layer 4 may contain components other than the metal oxides and SiO₂ to the extent that the sensitivity to a CO gas and response time are not significantly affected. The components other than the metal oxides and SiO₂ may constitute 5 vol% or less, in total, of the region of the sensing layer 4 excluding the pores.

The sensing layer 4 preferably has a low metal content, particularly a low Pt, Pd, Au, or Ag content, with easiness of decreasing raw material costs being considered. Specifically, the Pt, Pd, Au, or Ag content of the sensing layer 4 may be 5 mass% or less.

Hereinafter, structures of the metal oxide semiconductor gas sensor 10 according to the present embodiment and a method of manufacturing the same are described in more detail.

FIG. 1 shows the metal oxide semiconductor gas sensor 10 according to the present embodiment prior to formation of the sensing layer 4 (CO gas sensing layer). The first electrode 2 and the second electrode 3 are formed on the substrate 1.

Types of the substrate 1 are not limited. The substrate 1 may be, for example, a substrate composed of an insulator that is heat resistant to the operation temperature of the metal oxide semiconductor gas sensor 10. As the substrate 1, specifically, an electrically insulating ceramic substrate, a substrate with a thermal oxide film, or the like can be used. Examples of electrically insulating ceramic substrates include an alumina substrate and a zirconia substrate. Examples of substrates with a thermal oxide film include a silicon substrate with a thermal oxide film.

The substrate 1 is not an essential constituent of the metal oxide semiconductor gas sensor 10. It is only required that the metal oxide semiconductor gas sensor 10 include at least the first electrode 2, the second electrode 3, and the sensing layer 4 in contact with both the first electrode and the second electrode in a manner that enables the metal oxide semiconductor gas sensor 10 to exhibit its function as a gas sensor.

Materials of the first electrode 2 and the second electrode 3 are not limited. The first electrode 2 and the second electrode 3 are composed of an electrically conductive material. As a material of the first electrode 2 and the second electrode 3, for example, Pt, Au, or the like may be used.

Shapes of the first electrode 2 and the second electrode 3 are not limited. To increase the contact area with the sensing layer 4, the first electrode 2 and the second electrode 3 may have, for example, the interdigitated shape as shown in FIG. 1. In this situation, the contact area between the first electrode 2 and the second electrode 3 and the sensing layer 4 is readily increased, which is preferable. It is preferable for the first electrode 2 and the second electrode 3 to be disposed so as to face each other as shown in FIG. 1. Note that the distance between the first electrode 2 and the second electrode 3 is not limited. With a decrease in size of the metal oxide semiconductor gas sensor 10 being considered, the distance between the first electrode 2 and the second electrode 3 is preferably small. The distance may be, for example, 50 um or less.

Methods of forming the first electrode 2 and the second electrode 3 are not limited. They can be formed using, for example, a sputtering method, a vacuum deposition method, or a screen printing method.

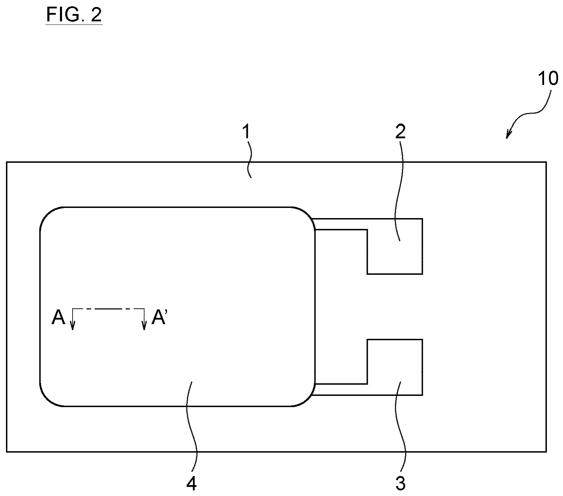

After the first electrode 2 and the second electrode 3 are formed, the sensing layer 4 may be formed on the first electrode 2 and the second electrode 3 as shown in FIG. 2. The shape, size, or the like of the sensing layer 4 are not limited. It is only required that the sensing layer 4 at least be in contact with both the first electrode 2 and the second electrode 3. The sensing layer 4 may be formed, for example, so as to cover the first electrode 2 and the second electrode 3 as shown in FIG. 2.

FIG. 3 is a sectional view along a line A-A' of FIG. 2. As shown in FIG. 3, the sensing layer 4 may be formed on the first electrode 2 and the second electrode 3 so as to fill a space between the first electrode 2 and the second electrode 3 in order to provide a sufficient contact area with the first electrode 2 and the second electrode 3.

Methods of forming the sensing layer 4 are not limited. The sensing layer 4 can be formed by, for example, turning In₂O₃ and Co₃O₄ into a paste together with a binder or the like, applying the paste to the substrate 1 having the first electrode 2 and the second electrode 3, and carrying out firing. At the time of firing, the binder or the like included in the paste may vaporize so that the sensing layer 4 contains In₂O₃ and Co₃O₄. At the time of firing, the firing temperature is preferably a temperature at which neither rapid grain growth nor necking among grains is facilitated. Rapid grain growth and necking of grains decrease the specific surface area necessary for a gas to adsorb on the sensing layer 4. Thus, specifically, the firing temperature (binder removal temperature) is preferably 350°C to 600°C. Note that the higher the firing temperature, the smaller φf - φn tends to be.

Methods of preparing the paste for forming the sensing layer 4 are not limited. The paste can be prepared by, for example, mixing and stirring In₂O₃ and Co₃O₄ with a vehicle made from the binder, an organic solvent, or the like. As the binder, for example, a binder composed of a polymer compound, such as cellulose, ethyl cellulose, or hydroxyethyl cellulose, can be used. As the organic solvent, toluene, xylene, terpineol, ethylene glycol, or the like can be used. The higher the binder content, the higher the porosity of the sensing layer 4 eventually obtained tends to be. The longer the mixing and stirring time, the smaller the average pore size tends to be.

For including the predetermined sensing layer 4, the metal oxide semiconductor gas sensor 10 of the present embodiment can have improved sensitivity to a CO gas and improved responsivity. Specifically, with high responsivity, the metal oxide semiconductor gas sensor 10 of the present embodiment can detect a CO gas at a low concentration (20 ppm or less) contained in a gas of interest. Note that there is no lower limit of the detectable concentration of a CO gas. A CO gas at a concentration of about 50 ppb or more can be detected.

Exposing the sensing layer 4, which is a semiconductor, to a CO-containing gas of interest changes the sensor resistance value, i.e., the value of resistance between the first electrode 2 and the second electrode 3. A CO gas can be detected from the change of the sensor resistance value.

Hereinafter, principles by which the sensor resistance value changes are described. For most metal oxide semiconductor gas sensors, a sensor resistance value at the time of exposure to air is used as a criterion. During exposure to air, electron-withdrawing oxygen is adsorbed on a surface of a semiconductor (sensing layer) of the metal oxide semiconductor gas sensors. In a situation where the semiconductor is an n-type semiconductor, adsorption of oxygen on the surface of the semiconductor forms a space charge layer in the vicinity of the surface of the semiconductor. Formation of the space charge layer forms a potential barrier between semiconductor particles and prevents electrons from moving between the semiconductor particles.

When an oxidizing gas flows into the surface of the semiconductor while oxygen is adsorbed on the surface of the semiconductor, the space charge layer further thickens. This results in an increase in the sensor resistance value. When a reducing gas flows into the surface of the semiconductor while oxygen is adsorbed on the surface of the semiconductor, the adsorbed oxygen is consumed to thin the space charge layer. This results in a decrease in the sensor resistance value. From above, the concentration of a gas of interest can be detected using the change of the sensor resistance value.

In a situation where the semiconductor is a p-type semiconductor, a reaction contrary to the reaction for an n-type semiconductor occurs. When an oxidizing gas flows into the surface of the semiconductor, the sensor resistance value decreases. When a reducing gas flows into the surface of the semiconductor, the sensor resistance value increases.

It is assumed that neither a potential barrier between n-type semiconductors nor a potential barrier between p-type semiconductors is applicable to the present embodiment. That is, it is assumed that, in the present embodiment, inside the sensing layer 4, In₂O₃ functions as an n-type oxide semiconductor, Co₃O₄ functions as a p-type oxide semiconductor, and an interface between these oxide semiconductors is provided with a p-n junction. Formation of the p-n junction combines an electron hole and an electron, forming an electron lacking layer that is wider and has a barrier higher than a conventional one at an interface of the junction to increase the resistance in air. Also, in a reducing gas or a flammable gas, because oxygen adsorbed on the surfaces of the oxides reacts with the gas and is partly consumed, captured electrons are back to the semiconductors to lower the space charge barrier to decrease the resistance. Consequently, it is assumed that the sensitivity is improved.

The sensitivity to a CO gas at a certain concentration is represented by Rg/Ra or Ra/Rg, where Ra denotes the sensor resistance value at the time of exposure of the metal oxide semiconductor gas sensor 10 to air not containing the CO gas, and Rg denotes the sensor resistance value at the time of exposure of the metal oxide semiconductor gas sensor 10 to a gas of interest containing the CO gas at the certain concentration. The sensitivity changes according to the concentration of the CO gas.

Ra > Rg is mostly satisfied when the metal oxide semiconductor gas sensor 10 including the sensing layer 4 according to the present embodiment is exposed to a CO gas.

It takes a certain amount of time for the sensor resistance value to change correspondingly after a gas being supplied to the metal oxide semiconductor gas sensor 10 is switched to another. Thus, during measurement of Ra, the sensor resistance value is continuously measured after supply of air to the metal oxide semiconductor gas sensor 10 has started to confirm stability of the sensor resistance value. The stabilized sensor resistance value is Ra. During measurement of Rg, the sensor resistance value is continuously measured after supply of the gas of interest to the metal oxide semiconductor gas sensor 10 has started to confirm stability of the sensor resistance value. The stabilized sensor resistance value is Rg.

Provided that the absolute value |(Ra - Rg)| of the amount of change of the sensor resistance value from Ra (sensor resistance value in air) to Rg (sensor resistance value in the gas of interest) is 100, the time it takes for the absolute value of the amount of change of the sensor resistance value to reach 90 after the gas being supplied to the metal oxide semiconductor gas sensor 10 is switched from air to the gas of interest is defined as a 90% response time (T90). R is represented by R = {(Ra - Rg) × 0.1 + Rg} (formula 1), where R denotes the sensor resistance value at the time when the 90% response time has passed. In other words, T90 is the time it takes for the sensor resistance value to change from Ra to R.

When a graph is to be plotted with time on the horizontal axis and the sensor resistance value on the vertical axis using a logarithmic scale because of a large difference between Ra and Rg, R' may be represented by logR' = {(logRa - logRg) × 0.1 + logRg} (formula 2), where R' denotes the sensor resistance value at the time when the 90% response time has passed. In this situation, T90 is the time it takes for the sensor resistance value to change from Ra to R'.

When the metal oxide semiconductor gas sensor 10 is exposed to the CO-containing gas of interest and then to air not containing the CO gas again, the sensor resistance value changes from Rg to Ra again. At this time, the time it takes for the sensor resistance value to change from Rg to Ra is preferably shorter. When the sensor resistance value in air does not stabilize and continues to increase or decrease during measurement, the sensor resistance value in the CO-containing gas of interest is larger or smaller than its proper value.

Thus, in a situation where Ra > Rg is satisfied, after the metal oxide semiconductor gas sensor 10 of the present embodiment is exposed to the CO-containing gas of interest and then to air not containing the CO gas again, the sensor resistance value preferably changes to a value that is 80% or more and 100% or less of Ra (sensor resistance value before exposure to the gas of interest) within five minutes. In a situation where Ra < Rg is satisfied, after the metal oxide semiconductor gas sensor 10 of the present embodiment is exposed to the CO-containing gas of interest and then to air not containing the CO gas again, the sensor resistance value preferably changes to a value that is 100% or more and 120% or less of Ra (sensor resistance value before exposure to the gas of interest) within five minutes.

The operation temperature of the metal oxide semiconductor gas sensor 10 of the present embodiment is not limited. The operation temperature is preferably, for example, 150°C or more and 400°C or less. This is because heating at 150°C or more can increase responsivity to the CO gas, which is a gas of interest. Heating at a high temperature exceeding 400°C may cause grain growth of In₂O₃ grains and/or Co₃O₄ grains, possibly degrading the metal oxide semiconductor gas sensor 10. To mitigate grain growth, prevent degradation or the like of the metal oxide semiconductor gas sensor 10, and enable its long-term use, the operating temperature is preferably 400°C or less as described above.

Methods of holding the metal oxide semiconductor gas sensor 10 at the above operating temperature are not limited. Any of various heating methods, such as external heating with an electric furnace or the like and resistance heating through formation of a heater (e.g., a Pt heater) on a substrate backside or the like and application of electricity to the heater, can be selected.

According to the metal oxide semiconductor gas sensor of the present embodiment, which has been described above, a CO gas contained at a concentration of about 20 ppm in a gas of interest can be detected at a small size and at low cost.

Types of the gas of interest are not limited. Any gases that contain a CO gas can be subject to measurement. In particular, a CO gas in a combustion engine exhaust gas can be suitably measured.

In particular, because the metal oxide semiconductor gas sensor of the present embodiment can detect a CO gas contained at a concentration of about 20 ppm with high sensitivity, the metal oxide semiconductor gas sensor can be suitably included in, for example, home healthcare applications.

Note that the present invention is not limited to the embodiment described above and can variously be modified. For example, displacement structures of the electrodes 2 and 3 of the sensor 10 are not limited. Any structures in which both the first electrode 2 and the second electrode 3 are in contact with the sensing layer and face each other are permissible.

Moreover, while In₂O₃ is used as a first metal oxide of an n-type semiconductor and Co₃O₄ is used as a second metal oxide of a p-type semiconductor in the embodiment described above, combinations are not limited. As a first metal oxide of another n-type semiconductor, for example, SnO₂, ZnO, WO₃, Al₂O₃, Fe₂O₃, TiO₂, or V₂O₅ may be used together with In₂O₃ or instead of In₂O₃. As a second metal oxide of another p-type semiconductor, NiO, FeO, Ag₂O, PdO, or the like may be used together with Co₃O₄ or instead of Co₃O₄.

### Examples

Hereinafter, the present invention is described in detail using examples; however, the present invention is not limited to these examples.

### (Examples 1 to 5, Comparative Examples 1 to 4)

A metal oxide semiconductor gas sensor 10 shown in FIGS. 1 to 3 was manufactured using the following procedure and was evaluated.

ED-IDE3-Au manufactured by MicruX Technologies molded into 9.5 × 5.0 mm² was prepared as a substrate 1.

On the substrate 1, electrodes 2 and 3 having an interdigitated shape shown in FIG. 1 were formed. Specifically, Pt electrodes having a size of 2.5 × 4.0 mm², an electrode width of 15 um, and a distance of 15 um therebetween in the interdigitated shape were formed using a sputtering method.

To the electrodes 2 and 3, a paste including a powder having a composition shown in Table 1 was applied. To apply the paste, an inkjet printer was used. Firing was carried out at a firing temperature of 430°C in air using a firing furnace to form a sensing layer 4 on the electrodes 2 and 3 as shown in FIG. 2. This manufactured the metal oxide semiconductor gas sensor 10.

A method of preparing the paste including the powder having the composition shown in Table 1 is described below.

An In₂O₃ powder (manufactured by Aldrich, average particle size 20 to 50 µm) and a Co₃O₄ power (manufactured by Aldrich, average particle size 20 to 50 µm) were mixed to give a mixed powder having the composition shown in Table 1. Separately, a polyvinyl butyral resin (BM-S manufactured by SEKISUI CHEMICAL CO., LTD.) and BDG (diethylene glycol monobutyl ether) were mixed to give a binder. They were weighed so that the resin mass ratio was 5 php with respect to the mixed powder. They were mixed and stirred using a room-temperature pulverizer (mixer mill) manufactured by SPEX for 1 hour to give the paste.

The paste, which was to become the sensing layer 4, was applied to the substrate 1 having the electrodes 2 and 3, and a heat treatment (400°C to 600°C) was carried out. This formed the sensing layer 4 including a single layer having a thickness of 1.2 µm to give the metal oxide semiconductor gas sensor sample.

A method of evaluating the manufactured metal oxide semiconductor gas sensor is described next.

The manufactured metal oxide semiconductor gas sensor was placed in a sample chamber with a heater. A sample holder with a heater was heated to 150°C to 400°C.

A nitrogen gas and an oxygen gas were mixed at a flow rate ratio of 4:1 to produce synthetic air. Synthetic air was introduced into the sample chamber at 500 mL/min to measure the sensor resistance value.

The sensor resistance value was measured with a two-terminal method using a 2700 multichannel DMM manufactured by Keithley Instruments. The sensor resistance value was measured at 10-second intervals. After stability of the sensor resistance value was confirmed, the sensor resistance value was measured at 10-second intervals and was averaged over 200 seconds. The resultant average value was deemed to be the sensor resistance value (Ra) for synthetic air.

After measurement of Ra, a certain amount of a CO gas was introduced into a nitrogen gas from a standard CO gas cylinder to produce a CO-containing gas. The CO-containing gas had a CO concentration of 20 ppm or less. The CO-containing gas was introduced into the sample chamber at 500 mL/min to supply the CO-containing gas to the metal oxide semiconductor gas sensor. A change in the sensor resistance value caused by the supply of the CO-containing gas was examined. The sensor resistance value measured after 15 minutes passed since the CO-containing gas started to be supplied was deemed to be Rg. Further, as sensitivity, Ra/Rg was calculated. Table 1 shows the results. An Ra/Rg of 2.00 or more was deemed good. An Ra/Rg of 2.50 or more was deemed better.

90% response time (T90) was identified using the above method. The time from when supply of the CO-containing gas started to when the sensor resistance value reached R' shown in the above formula 2 was deemed to be T90. A T90 of 120 seconds or less was deemed good. A T90 of 100 seconds or less was deemed better.

A backscattered electron image, taken with a scanning electron microscope (SU5000 manufactured by Hitachi, Ltd.), of a section of the sensing layer 4 parallel to its thickness direction was processed to calculate the average porosity of the section of the sensing layer 4. Further, the average pore size in the section of the sensing layer 4 was calculated. At this time, the backscattered electron image had a size that allowed the sensing layer 4 to be entirely observed in its thickness direction. The backscattered electron image was of a portion where the first and second electrodes would not be included in the image.

Further, the backscattered electron image was trisected in the thickness direction of the sensing layer 4. φf - φn was calculated, where φn (%) denoted the average porosity of a region closest to the first and second electrodes calculated by image processing of the backscattered electron image, and φf (%) denoted the average porosity of a region farthest from the first and second electrodes calculated by image processing of the backscattered electron image. Table 1 shows the results.

Using LA-ICP-MS, it was confirmed that the composition of a region of the sensing layer 4 excluding pores (the composition of the metal oxide material included in the sensing layer 4) was as shown in Table 1.

**[Table 1]**

| Sample No. | Composition [vol%] | | Sensing layer | Porosity difference | Average pore size | Sensing layer thickness | Sensitivity | Response time [T₉₀] |
|---|---|---|---|---|---|---|---|---|
| | In₂O₃ | Co₃O₄ | average porosity | φ_{f} - φₙ | [nm] | [um] | Ra/Rg | [sec] |
| Comparative Example 1 | ***100.0*** | ***0.0*** | 16.2% | 1.6% | 82 | 1.2 | ***1.63*** | ***123*** |
| Comparative Example 2 | **99.9** | **0.1** | 16.3% | 1.8% | 81 | 1.2 | ***1.79*** | ***120*** |
| Example 1 | 99.4 | 0.6 | 16.3% | 1.4% | 80 | 1.2 | 2.11 | 87 |
| Example 2 | 98.8 | 1.2 | 16.1% | 1.4% | 82 | 1.2 | 2.77 | 89 |
| Example 3 | 94.2 | 5.8 | 16.0% | 1.6% | 81 | 1.2 | 2.52 | 89 |
| Example 4 | 88.5 | 11.5 | 16.2% | 1.5% | 81 | 1.2 | 2.41 | 85 |
| Example 5 | 66.5 | 33.5 | 16.4% | 1.5% | 80 | 1.2 | 2.01 | 85 |
| Comparative Example 3 | ***46.0*** | ***54.0*** | 16.1% | 1.8% | 81 | 1.2 | 1.27 | 125 |
| Comparative Example 4 | ***0.0*** | ***100.0*** | 16.2% | 1.5% | 84 | 1.2 | ***0.87*** | 132 |
| Comparative Example 5 | 94.2 | 5.8 | ***15.5%*** | 0.8% | 76 | 1.2 | ***1.47*** | ***159*** |
| Example 3 | ***94*.*2*** | ***5.8*** | 16.0% | 1.6% | 81 | 1.2 | 2.52 | 89 |
| Example 6 | 94.2 | 5.8 | 16.9% | 1.3% | 82 | 1.2 | 3.70 | 84 |
| Example 7 | 94.2 | 5.8 | 20.8% | 1.9% | 85 | 1.2 | 5.08 | 82 |
| Comparative Example 6 | 94.2 | 5.8 | ***25.0%*** | 2.1% | 89 | 1.2 | ***Unm easurable*** | |
| Example 8 | 94.2 | 5.8 | 17.1% | 4.2% | 81 | 1.2 | 3.80 | 86 |
| Example 9 | 94.2 | 5.8 | 18.0% | 3.7% | 82 | 1.2 | 4.25 | 85 |
| Example 10 | 94.2 | 5.8 | 20.7% | 1.4% | 85 | 1.2 | 4.97 | 84 |
| Example 6 | 94.2 | 5.8 | 16.9% | 1.3% | 82 | 1.2 | 3.70 | 84 |
| Example 11 | 94.2 | 5.8 | 16.4% | 0.5% | 81 | 1.2 | 2.55 | 102 |
| Example 12 | 94.2 | 5.8 | 15.9% | 0.4% | 81 | 1.2 | 2.51 | 112 |
| Example 13 | 94.2 | 5.8 | 16.8% | 1.2% | 52 | 1.2 | 2.06 | 86 |
| Example 6 | 94.2 | 5.8 | 16.9% | 1.3% | 82 | 1.2 | 3.70 | 84 |
| Example 14 | 94.2 | 5.8 | 15.9% | 1.5% | 110 | 1.2 | 2.47 | 82 |
| Example 15 | 94.2 | 5.8 | 17.1% | 1.4% | 170 | 1.2 | 2.05 | 86 |
| Example 16 | 94.2 | 5.8 | 16.9% | 1.1% | 81 | 0.4 | 2.01 | 97 |
| Example 3 | 94.2 | 5.8 | 16.0% | 1.6% | 81 | 1.2 | 2.52 | 89 |
| Example 17 | 94.2 | 5.8 | 16.4% | 1.5% | 83 | 1.6 | 3.23 | 89 |
| Example 18 | 94.2 | 5.8 | 15.9% | 1.2% | 83 | 5.4 | 6.30 | 89 |
| Example 19 | 94.2 | 5.8 | 22.0% | 1.8% | 81 | 1.2 | 5.06 | 82 |
| Example 20 | 94.2 | 5.8 | 15.7% | 1.3% | 82 | 1.2 | 2.01 | 115 |

According to Table 1, Examples 1 to 5, in which the composition of the sensing layer 4 was within a predetermined range, had good sensitivity and good response time. By contrast, Comparative Examples 1 to 4, in which the composition of the sensing layer 4 was outside the predetermined range, did not have good sensitivity and/or good response time.

### (Examples 3 and 6 to 7, Comparative Examples 5 to 6)

The experiment was conducted as in Example 3 except that the resin content of the binder was changed from that of Example 3 to change the average porosity of the sensing layer 4 to a value shown in Table 1. Comparative Example 5, Example 3, Example 6, Example 7, and Comparative Example 6 were in ascending order of the resin content of the binder. The higher the resin content of the binder, the higher the average porosity. Table 1 shows the results.

Examples 3, 6, and 7, in which the sensing layer 4 had an average porosity of 15.7% or more and 22.0% or less, had good sensitivity and good response time. By contrast, Comparative Example 5, in which the average porosity was too low, did not have good sensitivity or good response time. In Comparative Example 6, in which the average porosity was too high, the sensing layer 4 had too low a shape-retaining property, and neither sensitivity nor response time was measurable.

### (Examples 8 to 10)

Two types of pastes with different binder contents were prepared. The paste having a smaller binder content was applied first, and then the other paste having a higher binder content was applied, so that φf - φn was eventually as shown in Table 1. The experiment was conducted as in Example 3 in other respects. Table 1 shows the results. Note that Examples 8, 9, and 10 were in descending order of the difference in the resin content of the binder between the paste for a first layer and the paste for a second layer. In Example 10, the difference in the resin content of the binder between the paste for the first layer and the paste for the second layer was 0.

Despite φf - φn being changed, each Example in which the composition and the average porosity of the sensing layer 4 were within the predetermined ranges had good sensitivity and good response time. Note that it is assumed that the average porosity of the sensing layer 4 affected the sensitivity and response time more than φf - φn did. It is assumed that that was why Example 10 had higher sensitivity and better response time than those of Examples 9 and 8. However, in any of Examples 8 to 9, sensitivity and response time were better than those of Comparative Example 5, in which φf - φn was less than 1.

### (Examples 6 and 11 to 12)

The experiment was conducted as in Example 6 except that the heat treatment temperature for forming the sensing layer 4 was increased so that φf - φn was eventually as shown in Table 1. Table 1 shows the results.

Despite φf - φn being changed, each Example in which the composition and the average porosity of the sensing layer 4 were within the predetermined ranges had good sensitivity and good response time. Note that, Example 6, in which φf - φn was 1.0 or more, had higher sensitivity and shorter response time than Examples 11 and 12, in which φf - φn was less than 1.0.

### (Examples 6 and 13 to 15)

The experiment was conducted as in Example 6 except that the mixing and stirring time during preparation of the paste was changed. Examples 13, 6, 14, and 15 were in descending order of the mixing and stirring time. The shorter the mixing and stirring time, the larger the average pore size. The average pore size was eventually as shown in Table 1. Table 1 shows the results.

Despite the average pore size being changed, each Example in which the composition and the average porosity of the sensing layer 4 were within the predetermined ranges had good sensitivity and good response time. Note that Examples 6 and 14, in which the average pore size was 80 nm or more and 120 nm or less, had better sensitivity and better response time than Example 13, in which the average pore size was less than 80 nm, and Example 15, in which the average pore size exceeded 120 nm.

### (Examples 16 to 18)

The experiment was conducted as in Example 3 except that the application thickness of the paste was changed to change the thickness of the sensing layer 4 as shown in Table 1. Table 1 shows the results.

Despite the thickness of the sensing layer 4 being changed, each Example in which the composition and the average porosity of the sensing layer 4 were within the predetermined ranges had good sensitivity and good response time. Note that Examples 3, 17, and 18, in which the sensing layer 4 had a thickness of 1.0 um or more, had better sensitivity and better response time than Example 16, in which the sensing layer had a thickness of less than 1.0 um.

### (Examples 19 and 20)

The experiment was conducted as in Example 3 except that at least one of the resin content of the paste, the application thickness, the heat treatment temperature, and the mixing and stirring time for the paste was changed to control the average porosity of the sensing layer as shown in Table 1. Table 1 shows the results. Examples 19 and 20, in which the sensing layer 4 had an average porosity of 15.7% or more and 22.0% or less, had good sensitivity and good response time.

## Claims

1. A metal oxide semiconductor gas sensor comprising:
a first electrode;
a second electrode; and
a sensing layer in contact with both the first electrode and the second electrode,
wherein
the sensing layer comprises In₂O₃ and Co₃O₄,
the sensing layer has an average porosity of 15.7% or more and 22.0% or less in a section of the sensing layer,
In₂O₃ constitutes 65 vol% or more and 99.5 vol% or less of a region of the sensing layer excluding pores, and
Co₃O₄ constitutes 0.5 vol% or more and 35 vol% or less of the region.

2. The metal oxide semiconductor gas sensor according to claim 1, wherein φf - φn ≥ 1.0 is satisfied, where φn (%) denotes an average porosity of a region closest to the first electrode and the second electrode, and φf (%) denotes an average porosity of a region farthest from the first electrode and the second electrode, provided that the sensing layer is divided into three regions in a thickness direction of the sensing layer.

3. The metal oxide semiconductor gas sensor according to claim 1, wherein the sensing layer has an average pore size of 80 nm or more and 120 nm or less in a section of the sensing layer.

4. The metal oxide semiconductor gas sensor according to claim 1, wherein the sensing layer has a thickness of 1.0 um or more.
